Europäisches Patentamt

European Patent Office

Office européen des brevets

(19)

(11) Publication number: **0 440 255 A1**

# EUROPEAN PATENT APPLICATION

(12)

(21) Application number: **91101366.2**

(22) Date of filing: **01.02.91**

(51) Int. Cl.5: **A61B 1/00**

(30) Priority: **01.02.90 JP 8125/90 U**

(43) Date of publication of application:
**07.08.91 Bulletin 91/32**

(84) Designated Contracting States:
**DE FR GB**

(71) Applicant: **MACHIDA ENDOSCOPE CO., LTD**
**13-8, Honkomagome 6-chome**
**Bunkyo-ku, Tokyo(JP)**

(72) Inventor: **Chikama, Toshio, c/o Machida**
**Endoscope Co.,Ltd.**
**13-8, Honkomagome 6-chome**
**Bunkyo-ku, Tokyo(JP)**

(74) Representative: **Koch, Günther, Dipl.-Ing.**
**Patentanwälte Wallach, Koch, Dr. Haibach,**
**Feldkamp et al**
**P.O. Box 121120**
**W-8000 München 12(DE)**

(54) **Endoscope cover.**

(57) An endoscope cover 6 comprising a covering member 6a which is a soft bag in which an intermediate conduit portion 3 is contained. In a closed face of the top end of the covering member 6a, at least a portion 6c,6d confronting an observation port and an illuminating port, arranged on the top end 5 of an endoscope 1, is made transparent. The entire length of the covering member is sufficient to cover at least the intermediate conduit portion 3. At least a top end rigid portion 5 of the endoscope 1 is bonded to the covering member 6a by an adhesive 7 so that the endoscope is covered with the cover member. An air-injecting opening 6f is formed at the rear end of the covering member.

Fig. 2

## ENDOSCOPE COVER

### Background of the Invention

The present device relate to a cover for covering the outer periphery of an endoscope when the endoscope is used.

After an endoscope is used in the body cavity of a patient, the endoscope is generally washed and disinfected. However, this washing and disinfecting operation requires much time and labor, and the working efficiency of the endoscope is very low. Furthermore, if this washing and disinfecting operation is not sufficiently performed, no satifactory washing and disinfecting effect can he attained.

Accordingly, an idea of a disposable cover for covering the outer periphery of an endoscope when the endoscope is used has recently been proposed, and an example of the disposable cover is disclosed in Japanese Unexamined Patent Publication No.61-179128.

The disclosed technique concerns an endoscope cover comprising a soft cylindrical coverning member composed of a rubber or synthetic resin, which is attached to rigid cover fitted to cover a top end rigid portion of the endoscope.

According to this endoscope cover, the rigid cover is fitted to the top end rigid portion of the endoscope, a flexible tube of the endoscope is entirely covered with the covering member from the top end side of the flexible tube, the endoscope is inserted in this state into the body cavity of a patient, and after the endoscope is used, the endoscope cover is dismounted and thrown away. When the endoscope is used again, the endoscope is covered with in new endoscope cover and is used in the above-mentioned manner. According to this technique, the endoscope need not be washed and disinfected, and a high sanitary effect is attained and the endoscope can he continuously used.

### (Problem to Be Solved by the Device)

When the above-mentioned endoscope is inserted into tile body cavity for the use, or is bent in the body cavity or repeatedly moved to and fro in the inserting direction, the rigid cover comes off from the top end rigid portion or the covering member gets out of position. Even if the rigid cover does not cone off, the field of vision is disturbed by the sippage of the rigid conver.

Furthermore, since both hands are used for the dismounting operation, the operation efficiency is low, and the dirty endoscope should be put at a certain place and the problem of contamination arises.

It is a primary object of the present invention to protect the intermediate conduit portion of the endoscope from contamination during use, and turns next use immediately after the use into posibility.

Another object of the present invention is to provide an endoscope cover, in which, the rigid cover is prevented from being taken off or getting out of position, and there can be attained an effect of ensuring complete covering.

Still another object of the present invention is to provide an endoscope cover, which can be taken out without contamination of the endoscope. This is another effect attained by the present device.

### Summary of the Invention

In accordance with the present device, the foregoing problems are solved by an endoscope cover comprising a covering member which is a soft bag in which an intermediate conduit portion is contained, wherein in a closed face of the top end of the covering member, at least a portion confronting an observation port and an illuminating port, arranged on the top end of an endoscope, is made transparent, the entire length of the covering member is fufficient to cover at least the intermediate conduit portion, and at least a top end rigid portion of the endoscope is bonded to the covering member by an adhesive so that the endoscope is covered with the cover member.

In the above-mentioned structure, the top end rigid portion of the endoscope is fitted and inserted into the covering member and all or a part of the peripheral surface of the top end conduit portion and intermediate conduit portion is bonded by an adhesive to the covering member, whereby the endoscope is entirely covered.

In this state, the intermediate conduit portion is inserted in the body cavity and used for the observation. After the use, by injecting air under pressure between the covering member and the intermediate conduit portion from the rear part of the covering member, the covering member is peeled from the intermediate conduit portion, and by pulling out the intermediate conduit portion in this state, the intermediate conduit portion can be drawn out from the covering member without contamination. By fitting a new endoscope cover to the used endoscope, the endoscope can be directly used again.

### Brief Description of the Drawings

Fig. 1 is a side view illustrating the state where an endoscope cover is attached to an endscope.

Fig. 2 is a perspective view of the endoscope cover.

Fig. 3 is a diagram illustrating the state where the endoscope cover is taken off from the endoscope.

Detailed Description of the Preferred Embodiments

An example of the present device will now be described with reference to the accompanying drawings.

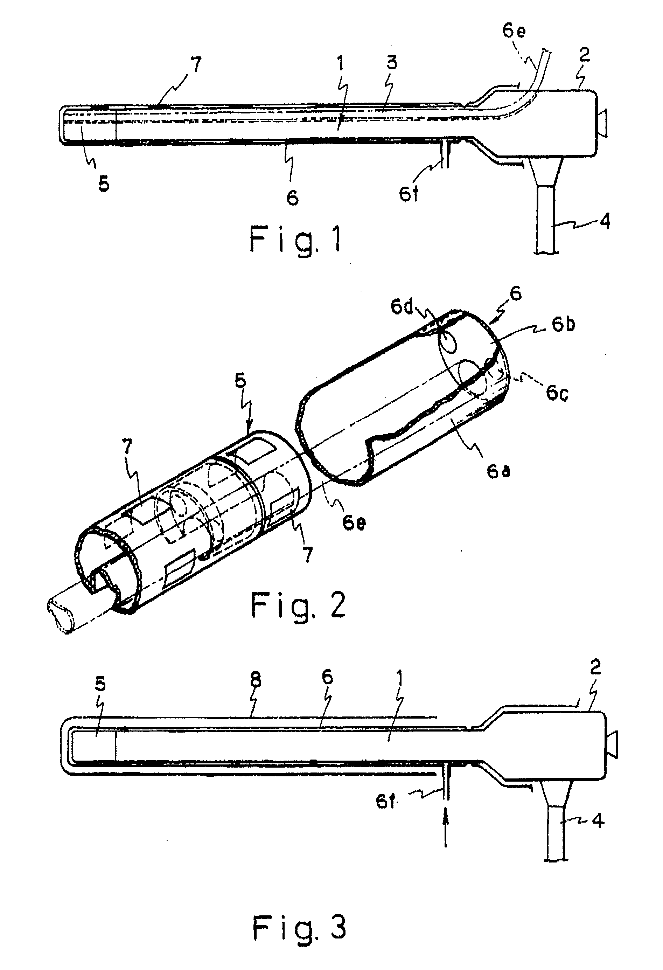

Fig. 1 is a side view illustrating the state where an endoscope cover is attached to an endoscope. Fig. 2 is a perspective view showing the endoscope cover. Fig. 3 is a diagram illustrating the state where the endoscope cover is taken off from the endoscope. In the drawings, reference numeral 1 represents an endoscope conrising a handle 2 an intermediate conduit portion 3 composed of a flexible tube and a guide tube portion connected to a light source.

In general, an image guide, a light guide and an angle-operating wire are disposed in the intermediate conduit portion 3.

The top portion of the intermediate conduit portion 3 forms a bendable angle portion, and a top end rigid portion 5 is attached to the top end of the angle portion.

An observation port connected to an image guide and an illuminating port connected to a light guide are formed on the top end of the top end rigid portion 5.

Reference numeral 5a represents a fitting groove in which a tube having both the ends opened, such as a forceps guide tube continuous from the intermediate conduit portion 3 to the top end rigid portion 5 or an air/water supply tube, is fitted.

Reference numeral 6 represents an endoscope cover comprising a covering member 6a which is a soft bag of a rubber or synthetic resin in which the intermediate conduit portion 3 is contained, Transparent portions 6c and 6d (both the transparent portions may be formed into one transparent portion) are formed on the top end closed face 6b of the covering member 6a at least at parts confronting the observation port and illuminating port which are formed on the top end of the endoscope, and the covering member 6a had a length sufficient to cover at least the intermediate conduit portion 3. If necessary, a hole corresponding to the above-mentioned fitting groove is formed on the closed face 6b and the end portion of the tube 6e having both the ends opened, such as the forceps guide tube, is attached to this hole. Of course, this hole need not be formed in case of an endoscope not using a forceps or the like, and in this case, the above-mentioned fitting groove and the tube having both the ends opened need not be formed.

The cylinder diameter of the endoscope cover 6 is adjusted to a value almost equal to or smaller than the outer diameter of the intermediate conduit portion 3 so that the endoscope cover 6 adheres closely to the intermediate conduit portion 3, or adjusted to a value slightly larger than the outer diameter of the intermediate conduit portion 3 so that the endoscope cover 6 loosely covers the intermediate conduit portion 3. In each case, the end portion of the covering member 6a adheres and anchors closely to the intermediate conduit portion 3 at the position of the root of the handle 2 or covers the handle 2 entirely. Reference numeral 6f represents an air-injecting opening.

In the example having the above-mentioned structure, an adhesive 7 is coated on all or a part of the outer peripheral surface of the top end rigid portion of the endoscope or an adhesive tape is applied there to, and the top end rigid portion 5 is fitted into the covering member 6a and the transparent portions 6c and 6d are positioning and fixed to confront the observation port and illuminating port.

Then, the covering member 6a is extended from the rear part along the intermediate conduit portion 3 and is partially or entirely bonded to cover the intermediate conduit portion 3 entirely or to cover even the handle 2 as well as the intermediate conduit portion 3. In this state, the intermediate conduit portion 3 is inserted into the body cavity and used for the observation.

After the observation, the endoscope cover is taken out from the endoscope. Namely, at first, as shown in Fig. 3, the endoscope is put into a transparent bag 8 having a length sufficient to contain the entire endoscope therein or contain at least the entire intermediate conduit portion 3 therein, so that the operation is carried out in the state where the endoscope is grasped through the bag 8.

At first, air is injected under pressure into the covering member 6a from the air-injecting opening 6e to peel the covering member 6a from the intermediate conduit portion 3 and separate the endoscope cover from the endoscope, and the intermediate conduit portion 3 can be drawn out from the endoscope cover by pulling it together with the bag 8. The endoscope cover is thrown away together with the bag 8, and therefore, the endoscope is not contaminated.

Accordingly, by attaching a new endoscope cover to the endoscope, the endoscope can be immediately used again.

As is apparent from tile foregoing detailed description, according to the present device, there is provided an endoscope cover comprising a covering member which is a soft bag in which an intermediate conduit portion is contained, wherein in a closed face of the top end of the covering member, at least a portion confronting an observation port and an illuminating port, arranged on the top end of an endoscope, is made transparent, the entire length of the covering member is sufficient to cover at least the intermediate conduit portion, and at least a top end rigid portion of the endoscope is bonded to the covering member by an adhesive so that the endoscope is covered with the cover member. In this structure, even if the endoscope is used, for example, in the body cavity, since the intermediate conduit portion, that is, the inserting portion, is covered with the covering member, the endoscope is not contaminated, and only by exchanging the endoscope cover with a new endoscope cover after the use, the endoscope can be immediately used again.

Moreover, since the top end rigid portion and intermediate conduit portion are bonded to the covering member of the endoscope cover, the endoscope cover is prevented from being taken off or getting out of position during the use, and there can be attained an effect of ensuring complete covering.

Furthermore, after the use, the covering member is peeled from the top end rigid portion and intermediate conduit portion by injecting air under pressure into the endoscope cover, and therefore, the endoscope cover can be taken out without contamination of the endoscope. This is another effect attained by the present device.

## Claims

1. An endoscope cover comprising a covering member which is a soft bag in which an intermediate conduit portion is contained, wherein in a closed face of the top end of the covering member, at least a portion confronting an observation port and an illuminating port, arranged on the top end of an endoscope, is made transparent, the entire length of the covering member is sufficient to cover at least the intermediate conduit portion, and at least a top end rigid portion of the endoscope is bonded to the covering member by an adhesive so that the endoscope is covered with the cover member.

2. An endoscope cover as set forth in claim 1, wherein an air-injecting opening is formed at the rear end of the covering member.

Fig. 1

Fig. 2

Fig. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB-A-2 218 636  (RHYS AP DELWYN PHILLIPS)<br>* page 2, line 15 - page 3, line 24 * * page 5, lines 16 - 33; figures * | 1 | A 61 B<br>1/00 |
| P,X | US-A-4 922 914  (E.O. SEGAL & W.R.SEGAL)<br>* column 3, lines 28 - 59 * * column 5, lines 6 - 50; figures 2, 5 * | 1 | |
| D,A | EP-A-0 184 778  (F.E. SILVERSTEIN & E.A. OPIE)<br>* page 10, line 15 - page 12, line 25 * | 1,2 | |
| A | US-A-4 878 485  (E.L. ADAIR)<br>* column 3, line 64 - column 5, line 44 * | 1,2 | |
| A | DE-A-3 233 564  (FUJI OPTICAL CO., LTD.)<br>* page 7, lines 5 - 26; figures 1-3 * * page 9, line 8 - page 10, line 27 * | 1,2 | |
|  |  |  | **TECHNICAL FIELDS SEARCHED (Int. Cl.5)**<br><br>A 61 B |

The present search report has been drawn up for all claims

| Place of search | Date of completion of search | Examiner |
|---|---|---|
| The Hague | 10 May 91 | RIEB K.D. |